# FASCICULE DE BREVET EUROPEEN

(11) **EP 1 377 371 B1**
(45) Date de publication et mention de la délivrance du brevet: **01.08.2007**
(21) Numéro de dépôt: 02730339.5
(22) Date de dépôt: 04.04.2002
(51) Int. Cl.: B01J 19/12, B01J 19/24, C07C 319/18, C07C 319/04

(54) **PROCEDE PHOTOCHIMIQUE SEMI-CONTINU ET DISPOSITIF POUR SA MISE EN OEUVRE**
SEMI-KONTINUERLICHES PHOTOCHEMISCHES VERFAHREN UND VORRICHTUNG ZUR DURCHFÜRUNG DESSELBEN
SEMI-CONTINUOUS PHOTOCHEMICAL METHOD AND DEVICE THEREFOR

(30) Priorité: 05.04.2001 FR 0104631
(43) Date de publication de la demande: 07.01.2004
(73) Titulaire: Arkema France, 92800 Puteaux (FR)
(72) Inventeur: HEBERT, Mélanie, F-64110 Jurancon (FR); OLLIVIER, Jean, F-64260 Arudy (FR); FREMY, Georges, F-64390 Sauveterre de Bearn (FR)
(74) Mandataire: Treuil, Claude
(86) Numéro de dépôt international: PCT/FR2002/001175
(87) Numéro de publication internationale: WO 2002/081080

(56) Documents cités:
- EP-A- 0 060 754
- EP-A- 0 967 202
- FR-A- 2 430 417
- FR-A- 2 700 714
- US-A- 5 037 618

## Description

La présente invention concerne le domaine des procédés photochimiques et a plus particulièrement pour objet un procédé photochimique réalisé en mode semi-continu, ainsi qu'un dispositif pour sa mise en oeuvre.

Par procédé réalisé en mode semi-continu, on entend ici un procédé dans lequel l'un au moins des réactifs est, dès le début de la réaction, introduit en totalité dans le réacteur photochimique alors que l'un au moins des réactifs est introduit progressivement dans le réacteur au fur et à mesure de sa consommation. Un tel mode opératoire s'avère parfois nécessaire pour respecter les conditions optimales de synthèse.

Dans un procédé photochimique opérant en milieu liquide, il est nécessaire que la partie rayonnante de la lampe soit constamment complètement immergée dans le milieu réactionnel liquide. Cette immersion totale est indispensable pour ne pas éclairer une phase gazeuse dont le comportement, sous irradiation ultra-violette, peut s'avérer dangereux ou gênant pour la réaction considérée. Ainsi, par exemple, dans la synthèse de sulfures et de mercaptans par réaction photochimique d'un alcène avec un mercaptan ou l'hydrogène sulfuré, l'irradiation UV de la phase gazeuse peut produire du soufre élémentaire qui est un inhibiteur bien connu des réactions radicalaires comme celles mises en oeuvre pour produire les sulfures ou les mercaptans. D'autre part, l'immersion complète de la partie rayonnante de la lampe permet également d'évacuer, par l'intermédiaire du milieu réactionnel, la chaleur émise par la lampe; cette disposition simplifie le procédé et minimise les équipements nécessaires (double enveloppe, circuit d'eau de refroidissement, échangeurs, etc.).

Cette disposition n'est cependant pas compatible avec la mise en oeuvre d'un procédé semi-continu où l'introduction progressive de l'un au moins des réactifs entraîne l'accroissement de volume du milieu réactionnel dans un réacteur de géométrie fixe.

Selon l'invention, ce problème a été résolu en menant le procédé photochimique dans un réacteur comprenant deux zones, la partie rayonnante de la lampe étant totalement immergée dans une première zone qui est complètement remplie de milieu réactionnel et se déverse par trop-plein dans une seconde zone dont le volume est suffisant pour contenir le volume de milieu réactionnel provenant de la première zone et correspondant sensiblement au volume du ou des réactif(s) introduit(s) progressivement.

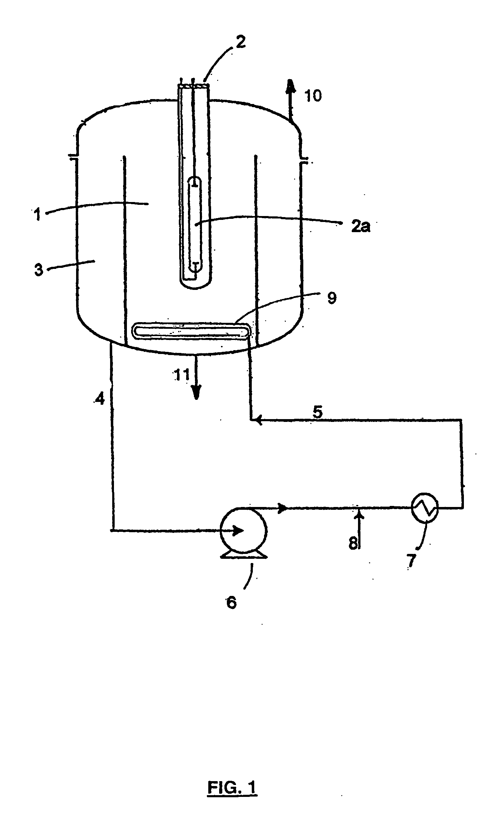

L'invention sera mieux comprise à la lumière du schéma de la figure 1 annexée qui montre les éléments essentiels d'un réacteur conforme à la présente invention. Ce réacteur comprend une zone réactionnelle (1) remplie complètement de milieu réactionnel au sein duquel est au moins totalement immergée la partie rayonnante (2a) d'une lampe UV (2) et, contiguë à la zone (1), une zone enveloppe (3) dans laquelle se déverse le trop-plein de la zone (1), des canalisations (4) et (5) permettant, via au moins une pompe (6) et un échangeur de chaleur (7), de recycler à la zone (1) par le distributeur (9) le milieu réactionnel après y avoir introduit par au moins une canalisation (8) le ou les réactif(s) additionnel(s), un évent de dégazage (10) et une canalisation (11) permettant de vidanger la zone (1) du réacteur.

Dans le mode de réalisation préféré tel que représenté à la figure 1, la zone cylindrique (1) et la zone enveloppe (3) sont coaxiales. Cependant, le concept inventif étant d'opérer dans un réacteur à deux zones, l'une remplie de milieu réactionnel dans lequel est totalement immergée la partie rayonnante de la lampe et l'autre recueillant le trop-plein de la première zone, on ne sortirait pas du cadre de la présente invention en travaillant dans un réacteur dont les deux zones (1) et (3) ne seraient pas coaxiales.

Conformément à la présente invention, la lampe (2) est installée dans la zone (1) de sorte qu'au moins toute la partie rayonnante (approximativement 65 % de la longueur de la lampe) soit immergée dans le milieu réactionnel, la partie supérieure (environ 35 %) correspondant à la partie électrique (câble d'alimentation) n'ayant pas besoin d'être immergée. Cependant, on ne sortirait pas du cadre de la présente invention en immergeant une plus grande partie de la lampe que celle correspondant à la partie rayonnante.

Comme indiqué précédemment, le volume de la zone (3) doit être suffisant pour contenir le volume de milieu réactionnel provenant de la zone (1) et correspondant sensiblement au volume du ou des réactif(s) introduit(s) progressivement. Le rapport entre les volumes des zones (1) et (3) dépend donc de la réaction considérée et pourra facilement être calculé par l'homme du métier à partir des paramètres réactionnels (volume de la zone 1, durée de la réaction, volumes des réactifs, etc...).

Le procédé et le dispositif selon l'invention ont été conçus pour la fabrication photochimique du méthyl éthyl sulfure (CH₃SCH₂CH₃) à partir d'éthylène et de méthyl mercaptan, mais ils sont également utiles non seulement pour la fabrication photochimique d'autres sulfures ou de mercaptans, mais aussi plus généralement pour toute synthèse photochimique nécessitant l'apport continu d'un ou plusieurs réactifs dans le réacteur au cours d'une opération.

La synthèse photochimique de sulfures et de mercaptans par réaction d'un alcène avec un mercaptan ou l'hydrogène sulfuré, éventuellement en présence d'un photo initiateur, s'effectue généralement avec une source lumineuse constituée par au moins une lampe au mercure basse pression ou toute autre source délivrant des radiations UV de longueurs d'onde comprises entre 200 et 400 nm. Cette réaction est de préférence réalisée sous une pression supérieure à la pression atmosphérique et pouvant aller de 0,1 à 50 bars relatifs selon l'alcène et le mercaptan (ou H₂S) de départ; pour la synthèse du méthyl éthyl sulfure, on opère de préférence sous une pression comprise entre 2 et 10 bars relatifs. Quant à la température réactionnelle, elle est généralement comprise entre -20 et 120°C (de préférence entre 10 et 90°C) et dépend non seulement du système de refroidissement, mais aussi de la pression de travail choisie.

Pour la fabrication du méthyl éthyl sulfure, le rapport du volume V1 de la zone (1) au volume V3 de la zone (3) est généralement compris entre 0,4 et 0,7.

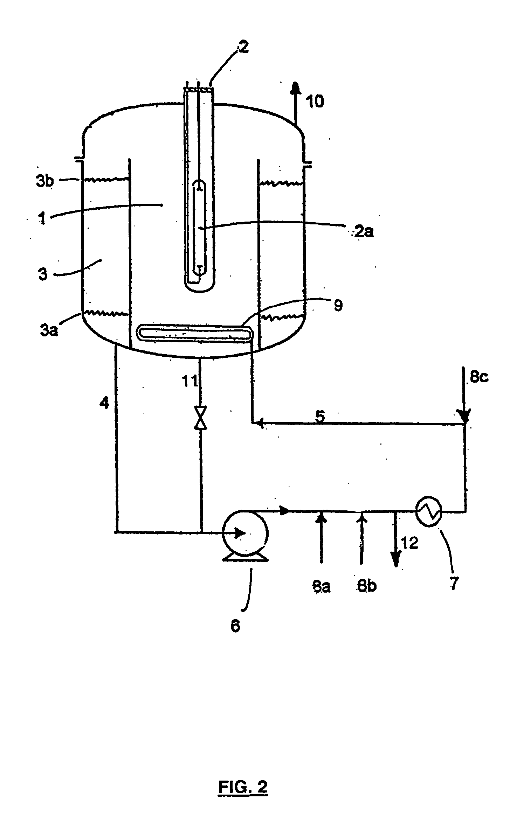

Le schéma de la figure 2 annexée représente un dispositif adapté à la fabrication en semi-continu de sulfures à partir d'un mercaptan et d'un alcène en présence d'un photo initiateur. Sur ce schéma, les entrées (8a), (8b) et (8c) sont respectivement celles du mercaptan, du photo initiateur et de l'alcène. L'opération consiste d'abord à remplir la zone (1) avec le mercaptan jusqu'à ce qu'il déborde dans la zone annulaire (3) à un niveau (3a) suffisant pour permettre l'amorçage de la pompe (6) utilisée pour assurer la circulation et l'agitation du milieu réactionnel. Après chargement du mercaptan par la canalisation (8a), la pompe (6) est mise en route et le liquide provenant de la zone (3) est refroidi dans l'échangeur (7), puis renvoyé vers la zone (1). Dès que la circulation est stable, la lampe (2) est allumée et, après quelques minutes de mise en régime, la réaction démarre dès l'introduction simultanée du photo initiateur par la canalisation (8b) et de l'alcène par la canalisation (8c). Le volume du milieu réactionnel augmentant au fur et à mesure de l'introduction du photo initiateur et de l'alcène, le niveau dans la zone (3) s'élève jusque, par exemple, au niveau (3b), mais la partie rayonnante de la lampe reste toujours totalement immergée dans le milieu réactionnel et refroidie en même temps par celui-ci. Quand on a fait réagir la quantité désirée de mercaptan, on arrête l'alimentation en alcène et photo initiateur, puis on éteint la lampe. Après dégazage par l'évent (10), le milieu réactionnel est recueilli par la canalisation (12) et éventuellement envoyé à une zone de purification qui, ne faisant pas l'objet de l'invention, n'est pas représentée ici.

L'exemple suivant illustre l'invention sans la limiter.

### EXEMPLE

Dans le dispositif décrit à la figure 2, on a préparé du méthyl éthyl sulfure en utilisant comme source lumineuse une lampe au mercure basse pression réémettrice par fluorescence des radiations voisines de 350 nm. Cette lampe de 58 watts était placée axialement dans la zone (1) du réacteur ayant une capacité totale de 50 litres, les volumes V1 et V3 étant respectivement de 15 et 35 litres.

On a chargé initialement 23,2 kg du méthyl mercaptan liquide de façon à remplir la zone (1) et à déborder dans la zone (3). On a introduit l'éthylène au débit de 1,03 kg/heure et le photo initiateur (2,2-diméthoxy-2-phénylacétophénone en solution à 200 g/l dans le méthyl éthyl sulfure) à un débit tel que la concentration en 2,2-diméthoxy-2-phénylacétophénone dans le milieu réactionnel soit de 0,1 g/litre. La pression dans le réacteur est montée de 3 à 9 bars absolus; la température était réglée à 45°C.

L'alimentation en éthylène et photo initiateur a été arrêtée après 13,1 heures et on a laissé la réaction se poursuivre en maintenant le milieu réactionnel en circulation pendant environ une heure.

La quantité de méthyl éthyl sulfure brut recueilli après détente dans le flux 12 était de 35,95 kg. A la pression atmosphérique et à la température ambiante, ce produit présentait la composition pondérale suivante :

| **Constituant** | **% poids** |
|---|---|
| Légers | 0,29 |
| Méthyl mercaptan | 0,001 |
| Ethyl mercaptan | 0,004 |
| Diméthyl sulfure | 0,07 |
| Méthyl éthyl sulfure | 99,57 |
| Diéthyl sulfure | 0,002 |
| Lourds | 0,06 |

L'effluent gazeux, sortant par 10, pesait 0,76 kg et présentait la composition pondérale suivante :

| **Constituant** | **% poids** |
|---|---|
| Légers | 39,3 |
| Méthyl mercaptan | 60,6 |

A partir de ces résultats, le rendement en méthyl éthyl sulfure s'établit à 97 % par rapport à l'éthylène et à 98 % par rapport au méthyl mercaptan.

## Revendications

1. Procédé de synthèse photochimique en mode semi-continu dans lequel l'un au moins des réactifs est introduit, dès le début de la réaction, en totalité dans le réacteur photochimique, le ou les autre(s) réactif(s) étant introduit(s) ensuite progressivement, **caractérisé en ce que** l'on opère dans un réacteur comprenant deux zones, la partie rayonnante de la ou des lampe(s) étant totalement immergée dans une première zone qui est complètement remplie de milieu réactionnel et se déverse par trop-plein dans une seconde zone dont le volume est suffisant pour contenir le volume de milieu réactionnel provenant de la première zone et correspondant sensiblement au volume du ou des réactif(s) introduit(s) progressivement.

2. Procédé selon la revendication 1 dans lequel le réactif initialement introduit en totalité dans la première zone est un mercaptan ou l'hydrogène sulfuré, le réactif introduit progressivement étant un alcène.

3. Procédé selon la revendication 2 dans lequel on introduit également progressivement un photo initiateur.

4. Procédé selon la revendication 2 ou 3 dans lequel on utilise une ou des lampe(s) délivrant des radiations UV de longueurs d'onde comprises entre 200 et 400 nm.

5. Procédé selon l'une des revendications 2 à 4 dans lequel on travaille sous une pression allant de 0,1 à 50 bars relatifs.

6. Procédé selon l'une des revendications 2 à 5 dans lequel on opère à une température comprise entre -20 et 120°C, de préférence entre 10 et 90°C.

7. Procédé selon l'une des revendications 2 à 6 dans lequel le mercaptan est le méthyl mercaptan et l'alcène est l'éthylène.

8. Procédé selon la revendication 7 dans lequel le rapport du volume de la première zone au volume de la seconde zone est compris entre 0,4 et 0,7.

9. Procédé selon la revendication 7 ou 8 dans lequel on opère sous une pression comprise entre 2 et 10 bars relatifs.

10. Dispositif pour la mise en oeuvre du procédé selon l'une des revendications 1 à 9, **caractérisé en ce qu'**il comprend essentiellement:
- une première zone 1 dans laquelle au moins une lampe 2 est placée de façon qu'au moins toute sa partie rayonnante 2a soit totalement immergée
- une seconde zone 3 contiguë à la zone 1 et lui servant de déversoir,
- des canalisations 4 et 5 et un distributeur 9 pour assurer, via au moins une pompe 6 et un échangeur de chaleur 7, la circulation du milieu réactionnel de la zone 3 vers la zone 1,
- au moins une canalisation 8 pour l'introduction des réactifs,
- un évent de dégazage 10, et
- une canalisation 11 de vidange.

## Claims

1. Photochemical synthetic process in semi-continuous mode in which at least one of the reagents is introduced, from the start of the reaction, in total into the photochemical reactor, the other reagent(s) then being introduced gradually, **characterized in that** the process is performed in a reactor comprising two zones, the radiating portion of the lamp(s) being totally immersed in a first zone which is completely filled with reaction medium and spills off via an overflow into a second zone whose volume is sufficient to contain the volume of reaction medium originating from the first zone and corresponding substantially to the volume of the reagent(s) gradually introduced.

2. Process according to Claim 1, in which the reagent initially introduced in total into the first zone is a mercaptan or hydrogen sulphide, the reagent gradually introduced being an alkene.

3. Process according to Claim 2, in which a photoinitiator is also introduced gradually.

4. Process according to Claim 2 or 3, in which one or more lamp(s) delivering UV radiation of wavelengths between 200 and 400 nm is(are) used.

5. Process according to one of Claims 2 to 4, which is performed at a pressure ranging from 0.1 to 50 bar relative.

6. Process according to one of Claims 2 to 5, which is performed at a temperature of between -20 and 120°C and preferably between 10 and 90°C.

7. Process according to one of Claims 2 to 6, in which the mercaptan is methyl mercaptan and the alkene is ethylene.

8. Process according to Claim 7, in which the ratio of the volume of the first zone to the volume of the second zone is between 0.4 and 0.7.

9. Process according to Claim 7 or 8, which is performed at a pressure of between 2 and 10 bar relative.

10. Device for carrying out the process according to one of Claims 1 to 9, **characterized in that** it essentially comprises:
- a first zone 1 in which at least one lamp 2 is placed such that at least all of its radiating portion 2a is totally immersed,
- a second zone 3 contiguous to the zone 1 and serving as an overspill therefor,
- pipes 4 and 5 and a distributor 9 for ensuring, via at least one pump 6 and a heat exchanger 7, circulation of the reaction medium from the zone 3 to the zone 1,
- at least one pipe 8 for introducing the reagents,
- a degassing vent 10, and
- a discharge pipe 11.

## Patentansprüche

1. Semi-kontinuierliches photochemisches Verfahren, bei dem mindestens eines der Reagentien von Beginn der Reaktion an in seiner Gesamtheit in den photochemischen Reaktor eingebracht ist, wobei das andere Reagens oder die anderen Reagentien dann nach und nach eingebracht werden, **dadurch gekennzeichnet, dass** in einem Reaktor mit zwei Zonen operiert wird, wobei der abstrahlende Teil der Lampe oder Lampen gänzlich in eine erste Zone getaucht ist, die vollständig mit Reaktionsmedium gefüllt ist und über einen Überlauf in eine zweite Zone ableitet, deren Volumen ausreicht, um das Reaktionsmilieuvolumen, das aus der ersten Zone kommt und im Wesentlichen dem Volumen des nach und nach eingebrachten Reagens bzw. der nach und nach eingebrachten Reagentien entspricht, zu fassen.

2. Verfahren nach Anspruch 1, bei dem das anfangs in seiner Gesamtheit in die erste Zone eingebrachte Reagens ein Mercaptan oder Schwefelwasserstoff ist, wobei das nach und nach eingebrachte Reagens ein Alken ist.

3. Verfahren nach Anspruch 2, bei dem auch ein Photoinitiator nach und nach eingebracht wird.

4. Verfahren nach Anspruch 2 oder 3, bei dem eine oder mehrere Lampe(n), die UV-Strahlung mit Wellenlängen im Bereich zwischen 200 und 400 nm abgeben, verwendet werden.

5. Verfahren nach einem der Ansprüche 2 bis 4, bei dem unter einem Druck von 0,1 bis 50 bar relativ gearbeitet wird.

6. Verfahren nach einem der Ansprüche 2 bis 5, bei dem bei einer Temperatur im Bereich zwischen -20 und 120 °C, vorzugsweise zwischen 10 und 90 °C, operiert wird.

7. Verfahren nach einem der Ansprüche 2 bis 6, bei dem das Mercaptan Methylmercaptan ist und das Alken Ethylen ist.

8. Verfahren nach Anspruch 7, bei dem das Verhältnis des Volumens der ersten Zone zum Volumen der zweiten Zone im Bereich zwischen 0,4 und 0,7 ist.

9. Verfahren nach Anspruch 7 oder 8, bei dem unter einem Druck im Bereich zwischen 2 und 10 bar relativ gearbeitet wird.

10. Vorrichtung zur Durchführung des Verfahrens nach einem der Ansprüche 1 bis 9, **dadurch gekennzeichnet, dass** sie im Wesentlichen aufweist :
- eine erste Zone 1, in der mindestens eine Lampe 2 derart angeordnet ist, dass mindestens ihr abstrahlender Teil 2a gänzlich eingetaucht ist,
- eine zweite Zone 3, die an die erste Zone 1 angrenzt und ihr als Überlauf-Auffang dient,
- Rohrleitungen 4 und 5 und einen Verteiler 9, um mittels mindestens einer Pumpe 6 und eines Wärmetauschers 7 die Zirkulation des Reaktionsmediums aus der Zone 3 in die Zone 1 sicherzustellen,
- mindestens eine Rohrleitung 8 für das Einbringen von Reagentien,
- einen Gasabzug 10, und
- eine Entleerungsleitung 11.
